# Europäisches Patentamt
# European Patent Office
# Office européen des brevets

(19)

(11) Numéro de publication: **0 050 562 B1**

(12) **FASCICULE DE BREVET EUROPEEN**

(45) Date de publication du fascicule du brevet:
28.03.84

(51) Int. Cl.³: **C 12 N 5/00**

(21) Numéro de dépôt: 81401605.1

(22) Date de dépôt: 15.10.81

(54) Procédé de production ou de biotransformation de métabolites par des cellules végétales in vitro.

(30) Priorité: 22.10.80 FR 8022538

(43) Date de publication de la demande:
28.04.82 Bulletin 82/17

(45) Mention de la délivrance du brevet:
28.03.84 Bulletin 84/13

(84) Etats contractants désignés:
AT BE CH DE FR GB IT LI LU NL SE

(56) Documents cités:
FR - A - 2 300 131
GB - A - 2 025 959
GB - A - 2 045 243

PROC. IV IFS: FERMENT. TECHNOL. TODAY, 1972 T.
FURUYA et al.: "Chemical constituents and
transformation capacity of medicinal plant callus
tissues", pages 705-709
CHEMICAL ABSTRACTS, vol. 93, 1980, ref. 120310t,
page 340, COLUMBUS, OHIO (US) P. BLANARIK et al.:
"Isolation of papaver somniferum callus tissue culture
and possibilities for the biosynthesis of opioid alkaloids
under in vitro conditions"
CHEMICAL ABSTRACTS, vol. 89, 1978, ref. 141144z,
page 122 COLUMBUS, OHIO (US) FURUYA TSUTOMU et
al.: "Biotransformation of (RS)-reticuline and morphinan

(72) Inventeur: Petiard, Vincent, 25, rue Pierre Valence
Champs Chardon, F-37100 Tours (FR)
Inventeur: Yvernel, Daniel, 4, Allée Marc Rebière St.
Cyr/Loire, F-37100 Tours (FR)

(74) Mandataire: Thouret-Lemaitre, Elisabeth, Service
Brevets - SYNTHELABO 58, rue de la Glacière,
F-75621 Paris Cedex 13 (FR)

(56) Documents cités: (suite)
alkaloids by cell cultures of papaver somniferum"
CHEMICAL ABSTRACTS, vol. 88, 1978, ref. 3119y, page
289 COLUMBUS, OHIO (US) KHANNA, PUSHPA et al.:
"Production of opium alkaloids from in vitro tissue
culture of Papaver rhoeas Linn"
CHEMICAL ABSTRACTS, vol. 87, 1977, ref. 130470k,
page 355, COLUMBUS, OHIO (US) T.I. ANDREEVA et al.:
"Indole alkaloids of a callus culture of Vinca rosea"
CHEMICAL ABSTRACTS, vol. 77, 1972, ref. 140362j, page
476, COLUMBUS, OHIO (US) T. FURUYA et al.: "Plant
tissue cultures. XV Alkaloids from callus tissue of
Papaver somniferum"
BIOLOGICAL ABSTRACTS, vol. 58, 1er août 1974, ref.
16578, PHILADELPHIA (US) UKITA, MASAYO et al.:
"5'-Phosphodiesterase formation by cultured plant
cells"
BIOLOGICAL ABSTRACTS, vol. 59, 15 mars 1975, ref.
33075, PHILADELPHIA (US) IKUTA, AKIRA et al.:
"Alkaloids of callus tissues and redifferentiated
plantlets in the Papaveraceae"

(73) Titulaire: SYNTHELABO, 58, rue de la Glacière,
F-75621 Paris Cedex 13 (FR)

**Procédé de production ou de biotransformation de métabolites par des cellules végétales *in vitro***

La présente invention a pour objet un procédé de production ou de biotransformation de métabolites par des cellules végétales. On entend par métabolites des produits de biosynthèse, de biotransformation ou de biodégradation réalisés par des cellules végétales. Ce procédé est caractérisé par le fait que ces cellules sont placées dans un milieu liquide non agité en l'absence de tout support de fixation ou d'inclusion, le milieu circulant sur les cellules sans les entraîner.

Les procédés de culture de cellules végétales, décrits dans la littérature, sont effectués dans des milieux où les cellules sont agitées et/ou maintenues sur des supports de fixation ou d'inclusion; par exemple dans les brevets britannique No 2045243, français No 2300131 et britannique No 2025959.

Il est connu que des cellules végétales cultivées *in vitro* peuvent, dans certains cas, biosynthétiser ou biotransformer des métabolites d'intérêt économique (Zenk M.H.: «The impact of plant cell culture on industry», *in* Thorpe T.A. (éd.), «The International Association for plant tissue culture», Calgary 1978, pp. 1-14).

L'exploitation industrielle de ce potentiel est actuellement limitée du fait du prix de revient des cultures de cellules végétales lorsqu'elles sont pratiquées dans des bioréacteurs traditionnels. Ce prix de revient beaucoup plus élevé que celui des procédés utilisant des micro-organismes est principalement dû à la durée des cultures, elle-même engendrée par la lenteur de prolifération de l'organisme.

A titre d'exemple, les taux de croissance maximale et les temps de génération de divers micro-organismes et de cellules végétales sont rapportés dans le tableau ci-dessous:

| | Bactérie | Levure | Champignon filamenteux | Cellule végétale |
|---|---|---|---|---|
| Temps de génération (tg) (h) | 0,3 | 1,5 | 3 | 24 |
| Taux de croissance maximale $\mu m$ ($h^{-1}$) | 2,3 | 0,46 | 0,23 | 0,0287 |

Le taux de croissance maximale est représenté par le rapport:

$$\frac{\Delta \text{ masse/masse}}{\text{temps}}$$

Cette comparaison a peu de sens sur le plan biologique, car elle ne tient pas compte de la taille des organismes. Cependant, sur un plan opérationnel, il est clair que la cellule végétale est l'organisme le plus défavorisé pour la réalisation de procédés biotechnologiques, du moins si la production recherchée est directement dépendante de la production de biomasse.

De ce fait, des procédés faisant appel à l'exploitation du milieu nutritif ont été étudiés, et il a clairement été montré (Petiard V., 1980, «Physiologie Végétale», 18, 2, 331-337) que certaines souches de tissus peuvent excréter les métabolites qu'elles ont biosynthétisés dans le milieu nutritif. Un de ces procédés consiste en la réalisation de réacteurs de cellules immobilisées soit par inclusion dans des gels d'alginate (Alfermann A.W., Schuler I. et Reinhard E., «Planta Medica», 1980, p. 281; Brodelius P., Deus B., Mosbach K., Zenk M.H., «Febs. Letters», 103, 1, 93-97), soit par fixation sur des supports divers.

L'objet de la présente invention est un procédé faisant appel au même type de réacteur, mais en l'absence de tout moyen d'immobilisation des cellules végétales.

Les cellules végétales sont placées sous forme d'agrégats cellulaires de taille variable (allant de la cellule isolée au cal) dans un milieu liquide non agité. Le milieu est alors mis en circulation pour assurer la nutrition, l'oxygénation et l'extraction éventuelle des métabolites excrétés. La biomasse reste décantée dans le réacteur grâce à un filtre de nature variable (verre fritté, toile d'acier inoxydable, toile de Nylon, etc.). La taille des agrégats permet, comparativement aux micro-organismes, de minimiser les phénomènes de colmatage.

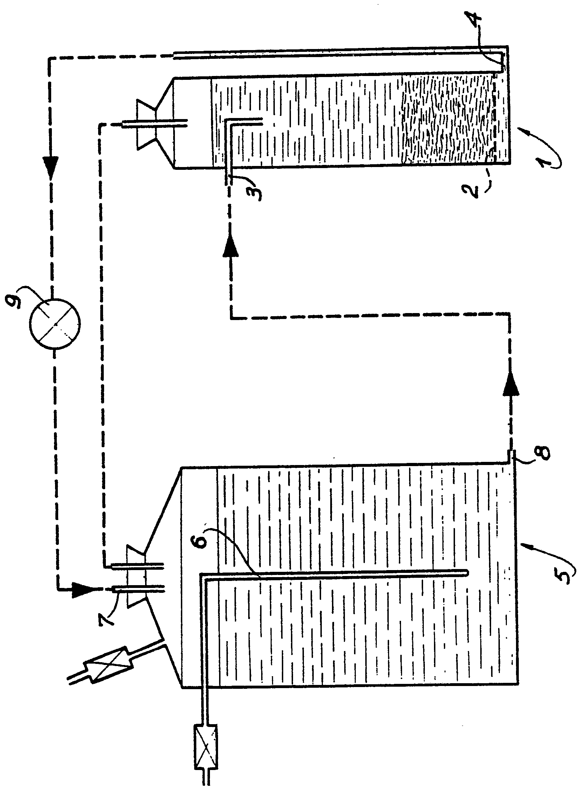

Sur la fig. 1 est représenté un modèle d'appareillage. Tout autre modèle permettant la culture de cellules végétales dans un milieu liquide non agité, et en l'absence de tout moyen de fixation ou d'inclusion des cellules, peut être utilisé pour la mise en œuvre de l'invention.

Le procédé de la présente invention peut être appliqué aux cellules végétales de l'ensemble des espèces de végétaux dits supérieurs ou phanérogames.

Les applications du procédé de la présente invention peuvent être aussi diverses que celles de procédés de cultures de cellules végétales en suspension dans des réacteurs classiques. Elles peuvent consister en la production de tous les composants du métabolisme primaire ou secondaire de la cellule végétale, par exemple, enzymes, essences, flaveurs, stéroïdes, pigments, alcaloïdes, flavones, hétérosides, composés nouveaux présentant des activités pharmacologiques intéressantes, etc. Ces applications peuvent également consister en la biotransformation spécifique de certains substrats en des composés précis recherchés pour des raisons diverses ou en la biodégradation de certains produits.

Le procédé de la présente invention peut donc trouver des applications dans des secteurs industriels divers: alimentaire, pharmaceutique, chimie pure, cosmétique, etc.

Il est possible de cultiver des tissus végétaux sous forme de cals décantés dans un milieu liquide, donc dans des conditions d'oxygénation limitées aux échanges de surface et, en conséquence, extrêmement réduites.

La description qui suit illustre ce fait.

*Obtention et multiplication de cals immergés de* Vinca minor L

Une de nos souches de tissus de *Vinca minor L* référencée Vm1 se présente sous forme de cals cultivés sur un milieu semi-solide gélosé de composition suivante:

Solution de macro-éléments minéraux dite de Heller

| Composants | Concentration en mg/l de milieu |
|---|---|
| Chlorure de potassium | 750 |
| Nitrate de sodium | 600 |
| Sulfate de magnésium heptahydraté | 250 |
| Phosphate monosodique monohydraté | 125 |
| Chlorure de calcium dihydraté | 75 |

Solution d'oligo-éléments minéraux dite de Heller

| Composants | Concentration en mg/l de milieu |
|---|---|
| Sulfate de zinc heptahydraté | 1 |
| Acide borique | 1 |
| Sulfate de manganèse tétrahydraté | 0,1 |
| Sulfate de cuivre pentahydraté | 0,03 |
| Chlorure d'aluminium hexahydraté | 0,05 |
| Iodure de potassium | 0,01 |
| Chlorure de nickel hexahydraté | 0,03 |

Source de fer
Chlorure ferrique hexahydraté 1 mg/l de milieu

Solution de vitamines référencée B1

| Composants | Concentration en mg/l de milieu |
|---|---|
| Panthoténate de calcium | 1 |
| Pyridoxine | 1 |
| Acide nicotinique | 1 |
| Thiamine | 1 |
| Méso-inositol | 10 |
| Biotine | 0,01 |

Substances de croissance
acide (dichloro-2,4 phénoxy)-2
acétique 1 mg/l de milieu
(furfurylamino)-6 purine
(kinétine) 1 mg/l de milieu
Substrat carboné: saccharose 20 g/l de milieu
Agent de solidification: gélose 7 g/l de milieu

Les cultures sont réalisées en tubes à essai et placées dans des conditions homogènes à une température de 23° C et sous un éclairement défini comme suit:
— spectre lumineux type lampe Sylvania Gro-lux
— intensité lumineuse de l'ordre de 5000 lux
— photo période: 16 h/d
— cycle de croissance: 4 semaines.

Dans ces conditions, les cals sont relativement chlorophylliens et très fermes, ils présentent un taux de croissance de 225% exprimé selon la formule:

$$\frac{PfC - Pfl}{Pfl} \times 100$$

$$\frac{\text{Poids de matière fraîche récoltée} - \text{Poids de matière fraîche de l'implant}}{\text{Poids de matière fraîche de l'implant}}$$

Leur teneur en sec est de 7,11%

$$\left(\frac{\text{matière sèche}}{\text{matière fraîche}} \times 100\right)$$

Lors d'un repiquage de cette souche, quelques cals ont été placés strictement dans les mêmes conditions, alors que d'autres cals ont été placés dans un milieu identique, à l'exception de l'agar qui a été supprimé. Il s'agit donc d'un milieu liquide (10 ml/tube à essai).

12 h environ après l'inoculation, les cals sont décantés au fond du tube à essai et recouverts par environ 2,5 à 3 cm de milieu liquide.

Après plusieurs repiquages dans ces conditions, des cals ayant présenté la meilleure croissance, on obtient une souche qui a un aspect sensiblement identique à celui de la souche cultivée dans les conditions initiales. Le taux de croissance de cette souche pour une même durée de culture est de 313%. Il est donc supérieur à celui de la souche initiale sur milieu gélosé. La teneur en matière sèche des tissus obtenus dans ces conditions est de 5,99%.

Les exemples suivants non limitatifs illustrent l'invention. D'une façon générale, en dehors du procédé de l'invention, les techniques classiques pour l'homme de l'art sont utilisées (stérilisation des milieux, conditions de culture, extraction, etc.).

*Exemple 1 :*

*Culture en colonne d'une souche de* Catharanthus roseus *G. Don référencée CR2 — Production en batch*

Une de nos souches, référencée CR2, de tissus de *Catharanthus roseus* présente une faible capacité de biosythèse et d'excrétion de métabolites, mais elle a été retenue pour son activité antimitotique (Petiard V., 1980, «International Research Congress on Natural Products as medicinal agents»).

Elle est classiquement cultivée en boîtes de Petri sur un milieu semi-solide ayant la composition suivante:

Solution de macro-éléments dite de Murashige et Skoog

| Composants | Concentration en mg/l de milieu |
|---|---|
| Nitrate d'ammonium | 1650 |
| Nitrate de potassium · | 1900 |
| Chlorure de calcium hydraté | 440 |
| Sulfate de magnésium heptahydraté | 370 |
| Phosphate de potassium | 170 |

Solution d'oligo-éléments dite de Heller (voir exemple 1)
Solution de vitamines référencée B1 (voir exemple 1)

| | |
|---|---|
| Chlorure ferrique hexahydraté | 1 mg/l |
| Substances de croissance | |
|    acide naphtyl-1-acétique | 1 mg/l |
|    (furfurylamino)-6 purine | 1 mg/l |
| Substrat carboné: glucose | 30 g/l |
| Agent de solidification: gélose | 7 g/l |

La souche est entretenue par repiquage régulier toutes les 4 semaines.

Des cals de cette souche âgés de 4 semaines sont placés stérilement dans un appareillage tel que celui de la fig. 1, contenant un milieu nutritif de composition identique, à l'exception de la gélose qui en a été supprimée. Cet appareillage comprend:

— Une colonne de culture 1 (de 3 l) comportant à sa partie inférieure un moyen de séparation des tissus et du milieu liquide, constitué par une plaque de verre fritté 2. Une tétine de verre 3 placée en haut de la colonne permet l'arrivée du milieu nutritif; une autre 4 placée en dessous de la plaque de verre fritté permet le départ du même milieu après circulation sur les tissus et sans risque d'entraînement de ces tissus.

— Une bonbonne de verre 5 (5 l) pouvant être thermostatée et pouvant recevoir divers moyens d'aération du milieu nutritif ou de mesure de certaines de ses caractéristiques (température, pH, densité d'oxygène dissous). Un moyen d'agitation 6 peut y être également ajouté afin d'assurer un meilleur transfert d'oxygène. Une tétine de verre 7 placée à sa partie supérieure permet le retour du milieu nutritif après passage sur la colonne de culture. Une autre tétine 8 placée à sa partie inférieure permet le départ du milieu vers la colonne de culture. Un moyen d'équilibrage de la pression entre les deux éléments principaux de cet appareillage peut également être adjoint.

— Une pompe 9 permet la mise en circulation du milieu entre ces deux récipients. Dans l'exemple présent, la pompe est placée sur le circuit de retour du milieu vers la bonbonne 2.

Le débit de circulation entre la colonne 1 et la bonbonne 2 est de 56 ml/min. La température du milieu est maintenue à 30°C au niveau de la partie inférieure de la colonne.

Les tissus sont restés sensiblement au même niveau chlorophyllien durant toute la durée de la culture.

Après 4 mois dans ces conditions, l'ensemble biomasse et milieu a été récolté.

Le poids de matière fraîche de biomasse a augmenté d'environ 100% (en 4 mois), ce qui représente une croissance relativement faible comparativement à la même souche cultivée sur milieu gélosé (1000% en 4 semaines).

La quantité d'extrait alcaloïdique brut obtenu par ce procédé est d'environ 95 mg: 50 mg à partir de la biomasse et 45 mg à partir du milieu liquide (5,6 mg/l × 8 l).

Cette quantité d'extrait alcaloïdique a donc été obtenue en 4 mois à partir d'un inoculum équivalent à la biomasse récoltée sur 10 boîtes de Petri.

En culture sur milieu gélosé, cette même biomasse produit 15 mg/mois (12 mg dans les tissus et 3 mg dans le milieu gélosé), soit 15 × 4 = 60 mg sur une période de 4 mois.

Globalement la production d'extrait alcaloïdique brut par le présent procédé est sensiblement 1,5 fois supérieure à celle obtenue dans les conditions de référence.

Il est à noter que la distribution tissus - milieu est différente dans les deux cas, et est plus favorable au milieu pour la culture en milieu liquide.

Qualitativement, la composition de cet extrait est sensiblement identique à celle d'extraits obtenus classiquement.

Certains composés alcaloïdiques aisément repérables par chromatographie en couche mince bidimensionnelle tels que l'ajmalicine, la serpentine, la (−)tabersonine, etc., y sont effectivement présents.

*Exemple 2:*

*Culture en colonne d'une souche de* Catharanthus roseus *G. Don référencée CR2. Production semi-continue ou continue*

La même souche que celle qui est décrite dans l'exemple 2 a été cultivée dans le même type d'appareillage auquel on a ajouté, au niveau de la bonbonne 5, un moyen de renouvellement semi-continu ou continu du milieu nutritif.

Dans le tableau 1 sont indiquées les quantités d'extrait alcaloïdique brut obtenues à partir du milieu nutritif lors de son renouvellement total après différentes durées de circulation sur la colonne de tissus.

La comparaison avec la production à partir de culture sur milieu gélosé est faite pour une quantité de tissus équivalente, c'est-à-dire pour la quantité récoltée à partir de 10 boîtes de Petri après 4 semaines de culture. Dans ces dernières conditions, on obtient 15 mg d'extrait réparti à raison de 12 mg dans les tissus et 3 mg dans le milieu gélosé.

Il ressort clairement de ce tableau:

— que la concentration dans le milieu est identique, quelle que soit la périodicité de renouvellement du milieu nutritif (de 1 à 4 semaines dans le présent exemple);

— que le coefficient d'amélioration du rendement par unité de temps et de biomasse, par rapport à la culture sur milieu solide, est d'autant plus élevé que la périodicité de renouvellement est faible; il doit être noté que cette comparaison ne prend en compte, pour le procédé décrit, que l'extrait obtenu à partir du milieu liquide.

L'intérêt du procédé est encore plus marqué si l'on compare cette production non plus à l'extrait de l'ensemble tissus - milieu des cultures sur milieu solide, mais seulement à l'extrait du milieu gélosé.

Pour cette souche la présence d'alcaloïdes dans le milieu semble donc due à un équilibre de diffusion. Dans ces conditions, le renouvellement total du milieu nutritif permet de ramener à 0 sa concentration en extrait alcaloïdique brut et, en conséquence, d'amplifier le phénomène de diffusion des alcaloïdes de la biomasse vers le milieu.

L'augmentation du volume de milieu en circulation sur une même quantité de biomasse ou le déplacement permanent de l'équilibre de concentration tissus - milieu sont des moyens qui permettent d'encore mieux exploiter ce phénomène de diffusion.

L'exemple 4 décrit un de ces procédés.

*(Tableau en page suivante)*

### Exemple 3:

*Culture en colonne d'une couche de* Catharanthus roseus *G. Don référencée CR2 — Extraction semi-continue ou continue des métabolites excrétés dans le milieu*

La même souche que celle qui est décrite dans les exemples 2 et 3 a été cultivée dans le même type d'appareillage auquel on a ajouté un moyen d'extraction continue des alcaloïdes contenus dans le milieu.

Ce moyen consiste en une colonne d'extraction remplie soit d'un solvant organique non miscible à l'eau (chloroforme, chlorure de méthylène, etc.), soit de résines permettant spécifiquement la fixation d'alcaloïdes (Amberlite XAD2 par exemple). Cette colonne d'extraction peut être placée soit directement sur le circuit de circulation du milieu, soit en parallèle à la bonbonne 5. Elle peut donc être utilisée soit de façon continue, soit sporadiquement (une fois par jour par exemple). Par ce procédé et pour les mêmes raisons que celles qui sont exposées dans l'exemple 3, le niveau de production dans le milieu liquide par unité de temps et de biomasse est fortement augmenté; il est multiplié par 10 ou par 40 selon que la comparaison prend ou non en compte l'extrait des tissus récoltés sur milieu gélosé. Contrairement au procédé décrit dans l'exemple 3, cette augmentation est obtenue sans renouvellement du milieu nutritif, et donc sans perte inutile des produits qui le composent. Cela est plus particulièrement important pour le substrat carboné dont la demande est relativement peu élevée, compte tenu des faibles taux de croissance de la biomasse cultivée dans ces conditions.

### Exemple 4:

*Culture en colonne d'une souche de* Catharanthus roseus *G. Don référencée CR20 — Production en batch, semi-continue, continue ou avec extraction continue*

Une autre de nos souches de *Catharanthus roseus* référencée CR20 est cultivée en suspension dans un milieu liquide agité. La composition de ce milieu nutritif est la suivante:

Solution de macro-éléments minéraux

| Composants | Concentration en mg/l de milieu |
|---|---|
| Nitrate de potassium | 2500 |
| Sulfate d'ammonium | 134 |
| Phosphate monosodique monohydraté | 150 |
| Sulfate de magnésium heptahydraté | 250 |
| Chlorure de calcium dihydraté | 150 |

Solution d'oligo-éléments minéraux

| Composants | Concentration en mg/l de milieu |
|---|---|
| Sulfate de manganèse monohydraté | 10 |
| Acide borique | 3 |
| Sulfate de zinc heptahydraté | 2 |
| Molybdate de sodium dihydraté | 0,250 |
| Sulfate cuivrique pentahydraté | 0,0250 |
| Chlorure de cobalt hexahydraté | 0,0250 |
| Iodure de potassium | 0,750 |

Solution de vitamines

| Composants | Concentration en mg/l de milieu |
|---|---|
| Méso-inositol | 100 |
| Acide nicotinique | 1 |
| Thiamine | 10 |
| Pyridoxine | 1 |

Source de fer
acide éthylènediaminotétraacétique dihydraté     37,3 mg/l
sulfate ferreux heptahydraté     27,8 mg/l
Substances de croissance
acide (dichloro-2,4 Phénoxy)-2
acétique     1 mg/l de milieu

Tableau 1

| Périodicité de renouvellement | Concentration en EC dans le milieu (mg/l) | EC total obtenu dans le milieu (pour 8 l) (mg) | Production d'EC par semaine (mg) | Production d'EC pour 4 semaines (mg) | Rapport de production ML/MS (milieu)/ (tissus+milieu) | Rapport de production ML/MS (milieu seul) |
|---|---|---|---|---|---|---|
| 1 semaine | 2,95 | 24 | 24 | 96 | 96/15 $\simeq$ 6,5 | 96/3 $\simeq$ 32 |
| 2 semaines | 3,10 | 24,8 | 14,4 | 57,6 | 57,6/15 $\simeq$ 4 | 57,6/3 $\simeq$ 19 |
| 3 semaines | 3,20 | 25,6 | 8,5 | 34 | 34/15 $\simeq$ 2,3 | 34/3 $\simeq$ 11 |
| 4 semaines | 3,80 | 30,4 | 7,5 | 30 | 30/15 $\simeq$ 2 | 30/3 $\simeq$ 10 |

ML = milieu liquide (en colonne)
MS = milieu solide (en boîtes de Petri)
EC = extrait brut

Production en milieu solide pour une même quantité de biomasse récoltée après 4 semaines de culture: 15 mg dont 12 mg dans les tissus et 3 mg dans le milieu.

(furfurylamino)-6 purine
(kinétine)                     0,06 mg/l de milieu
Substrat carboné: saccharose      20 g/l de milieu

Cette souche CR20 est cultivée en suspension dans des fioles d'Erlenmeyer agitées à une vitesse de 100-120 rotations/min et à une température de 26°C.

Elle est entretenue par repiquage régulier tous les 14 d dans du milieu nutritif neuf. Elle atteint dans ces conditions une densité maximale de 1,5 × 10⁶ cellules/ml. Elle se présente sous forme d'agrégats cellulaires très fins permettant un repiquage par simple prise d'une fraction aliquote.

Dans le présent exemple, cette souche est cultivée en deux phases successives:
— dans une première phase, elle est cultivée à 30°C dans un bioréacteur classique de 4,5 l utiles en milieu liquide agité et aéré; dans ces conditions, elle atteint un maximum de densité d'environ 250 g de matière fraîche par litre de culture; il s'agit donc d'une première phase de multiplication de la biomasse;
— dans une deuxième phase, la biomasse ainsi obtenue est placée dans un appareillage identique à celui décrit dans l'exemple 2 et représenté dans la fig. 1.

Très rapidement, l'ensemble de la biomasse décante sur la plaque de verre fritté de la colonne 1. La température de culture est maintenue à 30°C au bas de la colonne et le débit de circulation du milieu est également de 56 ml/min. Une aération de 0,1 V.V.M. (vol/vol/mm) est maintenue dans la bonbonne 5. Les tissus conservent une coloration blanc jaunâtre, aucun phénomène de brunissement caractéristique d'une nécrose n'apparaît durant toute la durée de la culture.

Après 4 mois de culture dans ces conditions, l'ensemble tissus - milieu est récolté. Le taux de croissance est de 100% environ. Il y a donc eu une faible croissance, que l'on avait d'ailleurs observée par une augmentation du niveau de la biomasse dans la colonne 1. La quantité d'extrait alcaloïdique brut obtenu dans ces conditions est, pour une quantité de tissus équivalente, environ 2 fois supérieure à celle qui a été obtenue à partir de cultures en milieu liquide agité (en fiole d'Erlenmeyer ou en réacteur) durant la même période. Qualitativement, ces extraits sont tout à fait similaires à ceux provenant de cultures réalisées classiquement. On peut, entre autres composés, y noter la présence d'ajmalicine et de serpentine.

Les diverses améliorations du procédé décrites dans les exemples 3 et 4 pour la souche CR2 ont également été mis en œuvre sur la souche C20. Ils ont augmenté d'environ 5 fois le rendement par unité de temps.

Des cultures de cellules végétales en milieu liquide non agité peuvent donc être réalisées également à partir de suspensions cellulaires développées dans des bioréacteurs classiques.

*Exemple 5:*

*Culture en colonne d'une souche de* Papaver somniferum *référencée PS 167*

Une de nos souches de *Papaver somniferum* référencée PS 167 est cultivée en tubes à essai sur un milieu semi-solide ayant la composition suivante:
— Solution de macro-éléments minéraux dite de Heller (voir exemple 1)
— Solution d'oligo-éléments minéraux dite de Heller (voir exemple 1)
— Chlorure ferrique hexahydraté
                              1 mg/l de milieu
— Solution de vitamines référencée B1 (voir exemple 1)
— Substances de croissance
    acide (dichloro-2,4- phénoxy)- acétique
                              0,1 mg/l de milieu
    (furfurylamino)-6 purine (kinétine)
                              1 mg/l de milieu
— Substrat carboné: glucose      30 g/l de milieu
— Agent de solidification: gélose   7 g/l de milieu

Les cultures sont placées dans les mêmes conditions que celles décrites pour la souche de *Vinca minor* dans l'exemple 1.

Les cals sont friables et relativement chlorophylliens. Cette souche a été retenue pour ses capacités de biosynthèse de certains alcaloïdes du pavot en dehors de tout phénomène de différenciation morphogène.

Avec des cultures de ce type âgées de 5 semaines, on inocule un appareillage tel que décrit dans l'exemple 2 et contenant un milieu liquide de composition identique à celle indiquée ci-dessus, à l'exception de la gélose qui en a été supprimée.

Morphologiquement, la culture conserve son aspect et ne montre pas de phénomènes de nécrose.

Le débit de circulation du milieu sur la colonne 1 est de 56 ml/min.

Après 3 mois de culture dans ces conditions, la biomasse et le milieu sont récoltés et extraits classiquement.

Le poids de matière fraîche récoltée est augmenté de 200% par rapport à celui de l'implant.

De même que dans les exemples précédents, le rendement en extrait brut alcaloïdique de la biomasse et du milieu est légèrement supérieur à celui observé pour les cultures en conditions classiques, c'est-à-dire sur milieu gélosé en tubes à essai.

Dans cet exemple également, l'augmentation de rendement est principalement due à la présence dans le milieu liquide en circulation sur les tissus de plus de 50% de l'extrait produit. La mise en œuvre des améliorations du procédé décrites dans les exemples 3 et 4 (renouvellement du milieu, extraction continue) accentue l'intérêt des cultures en milieu liquide non agité par rapport aux cultures sur milieu gélosé.

*Exemple 6:*

*Culture en colonne d'une souche de* Medicago sativa *référencée Ms CK2*

·Une de nos souches de *Medicago sativa* référencée Ms CK2 est cultivée sous forme d'une suspension cellulaire en fiole d'Erlenmeyer agitée.

Elle est cultivée à 27°C à l'obscurité et présente la particularité d'utiliser du lactose comme seule source de substrat carboné. Il a pu être montré, au cours d'études en fermenteur, que cette possibilité d'utilisation du lactose était due à deux activités β-galactosidasiques, une localisée dans les cellules et une localisée sur la paroi et dans le milieu nutritif.

Le milieu nutritif pour cette souche a la composition suivante:

— Solution de micro-éléments minéraux dite de Murashige et Skoog (voir exemple 2)
— Solution de micro-éléments minéraux dite de Heller (voir exemple 1)
— Source de fer (voir exemple 5)
— Solution de vitamines référencée B1 (voir exemple 1)
— Substance de croissance
  acide naphtyl-1-acétique    1 mg/l de milieu
  (furfurylamino)-6 purine    1 mg/l de milieu
— Substrat carboné: lactose    30 g/l de milieu

Dans le présent exemple, cette souche est cultivée en deux phases successives:
— dans une première phase, elle est cultivée à 27°C dans un fermenteur de 3,5 l utiles avec une agitation à pales de 80 rotations/min; la tension d'oxygène dissous du milieu est maintenue supérieure à 1,5 ppm; pour cela, le niveau d'aération est modulé de 0,03 à 0,5 V.V.M.; dans ces conditions, la culture atteint en 250 h une densité de 300 g de matière fraîche par litre;
— dans une deuxième phase, la biomasse ainsi obtenue est transférée dans la colonne 1 de l'appareillage décrit dans l'exemple 2 contenant le même milieu nutritif; dans ces conditions, les tissus décantent rapidement. La température est maintenue à 27°C en bas de la colonne 1 et le débit de circulation du milieu est fixé à 100 ml/min. Une aération de 0,25 V.V.M. est maintenue dans la bonbonne 5.

L'aspect morphologique de la culture reste identique pendant plus de 3 mois. Il a pu également être montré que, dans ces conditions, la culture conservait sa capacité d'hydrolyse du lactose (chute de la concentration en lactose du milieu et augmentation des concentrations en glucose et galactose) et que le milieu nutritif présentait une activité β-galactosidasique (activité O.N.P.G.).

Cet exemple montre que le procédé de la présente invention est également un moyen efficace de transformation enzymatique de certains substrats.

Des exemples non limitatifs décrits ci-dessus, il ressort clairement que le procédé de la présente invention permet de cultiver in vitro durant de longues périodes des cellules végétales en milieu liquide non agité et en l'absence de tout support artificiel d'inclusion ou de fixation de ces cellules et d'exploiter, dans ces conditions, certaines potentialités de leur métabolisme.

Le procédé de la présente invention permet:
— de maintenir des cultures en phase de production de certains métabolites durant des périodes très longues dépassant 4 à 6 mois;
— de faciliter fortement la production lorsqu'elle est, dans les conditions classiques, totalement déphasée par rapport à la croissance, car il permet aisément de freiner ou d'inhiber la croissance;
— d'exploiter au mieux les phénomènes de diffusion vers le milieu nutritif et, de ce fait, d'augmenter largement la production par unité de biomasse et par unité de temps;
— de réaliser une extraction continue des produits biosynthétisés et ainsi d'améliorer le rendement de production par rapport au substrat nutritif utilisé;
— d'exploiter des souches dont l'adaptation à une culture en milieu liquide agité se révèle impossible ou difficile, ou encore dont la capacité de production n'est exprimée qu'en présence de différenciations morphologiques apparaissant uniquement au niveau des cals.

Par sa simplicité, le procédé de la présente invention présente également de nombreux autres avantages par rapport aux procédés classiques de culture en milieu liquide agité: il limite les risques de contamination, les coûts d'utilisation (énergie, quantité de milieu consommée) et d'amortissement (simplicité du matériel).

Pour ces différentes raisons, le procédé de la présente invention est donc susceptible d'être utilisé dans de nombreuses applications pour la production ou la biotransformation de substances naturelles d'origine végétale, et cela de façon compétitive avec l'extraction de la plante entière ou la synthèse organique.

## Revendications

1. Procédé de production ou de biotransformation de métabolites par des cellules végétales in vitro, caractérisé par le fait que les cellules sont cultivées en milieu liquide non agité et en l'absence de tout support artificiel d'inclusion ou de fixation de ces cellules, le milieu circulant sur les cellules sans les entraîner.

2. Procédé selon la revendication 1, caractérisé par le fait que l'on utilise comme inoculum des cals de cellules végétales.

3. Procédé selon la revendication 1, caractérisé par le fait que l'on utilise comme inoculum des suspensions cellulaires cultivées en fermenteur.

4. Procédé selon l'une des revendications 1 à 3, caractérisé par le fait que l'on renouvelle en semi-continu ou en continu le milieu nutritif utilisé.

5. Procédé selon l'une des revendications 1 à 4, caractérisé par le fait que l'on extrait en continu les métabolites produits.

6. Procédé selon l'une des revendications 1 à 5, caractérisé par le fait qu'on limite partiellement ou totalement la croissance de la biomasse par suppression d'un élément du milieu nutritif.

7. Procédé selon l'une des revendications 1 à 6, caractérisé par le fait qu'il est utilisé pour la culture de tissus ou de cellules de végétaux appartenant à l'embranchement des phanérogames.

## Claims

1. Process for the production or biotransformation of metabolites by plant cells in vitro, which process is characterised in that the cells are cultivated in a non-agitated liquid medium and in the absence of any artificial carrier for including or fixing these cells, the medium circulating on the cells without carrying them away.

2. Process according to claim 1, characterised in that calluses of plant cells are used as the inoculum.

3. Process according to claim 1, characterised in that cell suspensions cultivated in a fermentor are used as the inoculum.

4. Process according to any one of claims 1 to 3, characterised in that the nutrient medium used is renewed semi-continuously or continuously.

5. Process according to any one of claims 1 to 4, characterised in that the metabolites produced are continuously removed from the liquid medium.

6. Process according to any one of claims 1 to 5, characterised in that the growth of the biomass is partially or totally limited by omitting a constituent of the nutrient medium.

7. Process according to any one of claims 1 to 6, characterised in that it is used for the cultivation of tissues or plant cells belonging to the family of the phanerogams.

## Patentansprüche

1. Verfahren zur Herstellung oder zur Biotransformation von Metaboliten mittels Pflanzenzellen in vitro, dadurch gekennzeichnet, dass die Zellen in einem flüssigen, nicht gerührten Medium in Abwesenheit jedes künstlichen, zum Einschluss oder zur Fixierung der Zellen dienenden Trägers gezüchtet werden und dass das Medium über den Zellen zirkuliert, ohne sie mitzunehmen.

2. Verfahren nach Anspruch 1, dadurch gekennzeichnet, dass man als Inoculum Kallusse von Pflanzenzellen verwendet.

3. Verfahren nach Anspruch 1, dadurch gekennzeichnet, dass man als Inoculum im Fermenter gezüchtete Zellsuspensionen verwendet.

4. Verfahren nach irgendeinem der Ansprüche 1 bis 3, dadurch gekennzeichnet, dass man das verwendete Nährmedium in halbkontinuierlicher oder kontinuierlicher Weise erneuert.

5. Verfahren nach irgendeinem der Ansprüche 1 bis 4, dadurch gekennzeichnet, dass man die gebildeten Metabolite kontinuierlich extrahiert.

6. Verfahren nach irgendeinem der Ansprüche 1 bis 5, dadurch gekennzeichnet, dass man das Wachstum der Biomasse teilweise oder zur Gänze durch Weglassung eines Elementes des Nährmediums beschränkt.

7. Verfahren nach irgendeinem der Ansprüche 1 bis 6, dadurch gekennzeichnet, dass es für die Züchtung von Geweben oder Zellen von Pflanzen verwendet wird, die zum Zweig der Phanerogamen gehören.